# EUROPÄISCHE PATENTANMELDUNG

(11) **EP 1 020 181 A1**
(43) Veröffentlichungstag der Anmeldung: **19.07.2000**
(21) Anmeldenummer: 99125697.5
(22) Anmeldetag: 23.12.1999
(51) Int. Cl.: A61K 9/16

(54) **Verfahren zur Herstellung von Pellets mit einem Gehalt von bis zu 90 Gew. % eines pharmazeutischen Wirkstoffes**

(30) Priorität: 18.01.1999 DE 19901692
(71) Anmelder: Grünenthal GmbH, 52078 Aachen (DE)
(72) Erfinder: Bartholomäus, Johannes Heinrich, Dr., 52080 Aachen (DE); Ziegler, Iris, Dr., 52159 Rott-Roetgen (DE)

(57) **Zusammenfassung**

Die vorliegende Erfindung betrifft Verfahren zur Herstellung von Pellets enthaltend ≥ 50 Gew.% eines pharmazeutischen Wirkstoffs mit einer Wasserlöslichkeit ≥ 0,5 g/ml durch wässrige Granulation der wirkstoffhaltigen Mischung, Extrusion, Rundung und Trocknung der feuchten Granulate, indem eine wirkstoffhaltige Mischung eingesetzt wird, die aus
A) wenigstens 50 Gew.% wenigstens eines Wirkstoffes mit einer Wasserlöslichkeit von ≥ 0,5 g/ml
B) höchstens 50 Gew.% der Kombination aus
   a) einer mikrokristallinen Cellulose mit einer mittleren Teilchengröße im Bereich von 15 - 25 µm
   und
   b) einer niedrig substituierten Hydropropylcellulose mit einer mittleren Teilchengröße im Bereich von 10 - 25 µm
   besteht,
wobei das Gewichtsverhältnis von a) : b) im Bereich von 4:6 bis 6:4 liegt.

## Beschreibung

Die vorliegende Erfindung betrifft Verfahren zur Herstellung von Pellets rnit einem Gehalt von bis zu 90 Gew.% eines pharmazeutischen Wirkstoffes mit extrem hoher Wasserlöslichkeit durch wässrige Feuchtextrusion und anschließende Spheronisation.

Die Extrusion und anschließende Spheronisation ist eine seit langem bekannte Methode zur Herstellung von Granulaten mit definierter Form und Korngröße, die auch große Bedeutung für die Herstellung pharmazeutischer Pellets gewonnen hat (J.W. Conine et al., Drug & Cosmetic ind. 106, 38-41 (1970)). Dabei ist auch die Verarbeitung zahlreicher pharmazeutischer Wirkstoffe beschrieben worden, da solche multipartikulären Darreichungsform wegen einer besseren Bioverfügbarkeit, Arzneimittelsicherheit und Zuverlässigkeit der Wirkung oft der monolithischen Arzneiform vorgezogen werden. Darüber hinaus existiert umfassende Literatur, die sich mit der Optimierung der Herstellungsparameter, dem Einfluß der Rezepturzusammensetzung und den Unterschieden zwischen verschiedenen Extrudertypen, sowie mit den Grundlagen der Extrusion/Spheronisation beschäftigt (L. Hellen et al., Int. J. Pharm, 95, 197-204 und 205-216 (1993); L. Baert et al. Int. J. Pharm, 96, 225-229 (1993) and Int. J. Pharm., 81, 225-233 (1992) and Int. J. Pharm., 97, 79-92 (1993); K. Thoma et al. Drug Dev. Ind. Pharm., 24 (5), 401-411 (1998)).

Der Vorteil der Extrusion/Spheronisation zur Herstellung von Pellets gegenüber der Aufbaupelletisierung besteht u. a. in einer höheren Verdichtung der Pellets. Deshalb lassen sich mit der genannten Methode homogene Pellets mit hoher Wirkstoffbeladung, d. h. einer Wirkstoffbeladung bis zu 90 Gew.% erzielen. Zudem sind die durch Extrusion/Spheronisation hergestellten Pellets nicht nur dichter, sondern weisen auch eine geringere Porösität der Pelletoberfläche auf, so daß die Auftragsmenge für funktionelle Filme deutlich reduziert werden kann und gleichmäßigere Freisetzungsprofile erzielt werden können (G. Zhang et al., Drug Dev. Ind. Pharm., 16 (7), 1171-1184 (1990)). Daher ist die Herstellung von Pellets, insbesondere für die Herstellung von hochdosierten Pellets, die mit Retardüberzügen versehen werden, durch Extrusion/Spheronisation, bevorzugt.

Neben der sogenannten Schmelzextrusion ist die Extrusion mit Wasser angefeuchteter Granulate eine der häufigsten Extrusionsmethode. Die Wirkstoffe werden dabei gemeinsam mit den Hilfsstoffen unter Zusatz von Wasser granuliert und anschließend extrudiert, bevor die Extrudate in einem Spheroniser gerundet und getrocknet werden. Diese Methode hat gegenüber der Schmelzextrusion den Vorteil, daß unerwünschte Wärmebelastung der Wirkstoff-haltigen Mischungen vermieden wird.

Während durch die Schmelzextrusion Pellets mit einer bis zu 90 Gew.% hohen Wirkstoffbeladung auch für gut bis sehr gut wasserlösliche Wirkstoffe (WO96/14059) hergestellt werden können, hängt die Grenze der Wirkstoffbeladung von Pellets, hergestellt nach der wässrigen Extrusionsmethode ganz entscheidend vom Ausmaß der Wasserlöslichkeit des Wirkstoffes ab. So werden z. B. für wenig bis schlecht wasserlösliche Wirkstoffe Pellets mit Wirkstoffgehalten über 80 Gew.% beschrieben, wobei die Pellets trotz des geringen Hilfsstoffanteils noch ausreichend rund sind und eine enge Korngrößenverteilung aufweisen können (G.A. Hileman et al., Drug Dev. Ind. Pharm., 19(4), 483-491 (1993)). Anerkanntes Fachwissen ist jedoch, daß je besser die Wasserlöslichkeit der Wirkstoffe ist, desto weniger Wirkstoff kann in die Pellets eingebaut werden (J.M Newton et al., Pharm. Research, 509-514 (1998); P.H. Harrison, J. Pharm. Pharmacol., 37, 686-691 (1985)). Dementsprechend sind für gut wasserlösliche Wirkstoffe mit einer Wasserlöslichkeit bis 0,3 g/ml in der Regel nur Pellets mit einem Wirkstoffgehalt von maximal 60 Gew.% mit Hilfe der wässrigen Feuchtextrusion gut herstellbar.

Aufgabe der vorliegenden Erfindung war es daher, Pellets von sehr gut wasserlöslichen, pharmazeutischen Wirkstoffen, d. h. solchen mit einer Wasserlöslichkeit ≥ 0,5 g/ml, vorzugsweise ≥ 1 g/ml, zur Verfügung zu stellen, die mit Hilfe des wässrigen Feuchtextrusionsverfahrens hergestellt werden und vorteilhafterweise nicht nur eine definierte Korngröße und sehr gute Rundung, sondern auch ein relativ enges Kornspektrum aufweisen.

Diese Aufgabe wird durch die erfindungsgemäßen Verfahren zur Herstellung von Pellets enthaltend ≥ 50 Gew.% eines pharmazeutischen Wirkstoffs mit einer Wasserlöslichkeit ≥ 0,5 g/ml durch Granulation der wirkstoffhaltigen Mischung mit Wasser, Extrusion, Rundung und Trocknung der feuchten Pellets gelöst, die dadurch gekennzeichnet sind, daß die wirkstoffhaltige Mischung aus
A) wenigstens 50 Gew.%, vorzugsweise wenigstens 65 Gew.%, wenigstens eines Wirkstoffs mit einer Wasserlöslichkeit > 0,5 g/ml, vorzugsweise > 1 g/ml, und
B) höchstens 50 Gew.%, vorzugsweise höchstens 35 Gew.% der Kombination aus
   a) mikrokristalliner Cellulose mit einer mittleren Teilchengröße von 15 - 25 µm bestimmt durch Laserbeugung (Malvern Master Sizer)
   und
   b) niedrig substituierter Hydroxypropylcellulose mit einer mittleren Teilchengröße im Bereich von 10 - 25 pm, gemessen mit Hilfe der Laserbeugung, besteht,
wobei das Gewichtsverhältnis von a) : b) im Bereich von 4:6 bis 6:4 liegt, und in die Mischung nur soviel Wasser eingearbeitet wird, daß diese ausreichende Plastizität zur Extrusion und Sphäronisation hat.

Mit dem erfindungsgemäßen Verfahren können Pellets hergestellt werden, die bis zu 90 Gew.% eines Wirkstoffes mit extrem hoher Wasserlöslichkeit, d. h. einer Wasserlöslichkeit von wenigstens 0,5 g/ml, wie z. B. Tramadolhydrochlorid (> 3,0 g/ml), Chlorpromazinhydrochlorid (2,5 g/ml), Metamizol-Na (>1 g/ml), Diphenhydraminhydrochlorid (860 mg/ml) hergestellt werden.

Für die erfindungsgemäße Herstellung ist wesentlich, daß die zum Einsatz kommenden Hilfsstoffe, nämlich mikrokristalline Cellulose und niedrig substituierte Hydroxypropylcellulose, eine bestimmte mittlere Teilchengröße haben und in einem bestimmten Gewichtsverhältnis zueinander eingesetzt werden. So ist eine mikrokristalline Cellulose mit einer mittleren Teilchengröße von 15 - 20 µm, wie z. B. Avicel™PH105 oder Emcocel SP 15™, oder niedrig substituierte Hydroxypropylcellulose mit einer mittleren Teilchengröße im Bereich von 10 - 25 µm, wie z. B. I-HPC LH 31^{TM}, I-HPC LH 32^{™} oder I-HPC LH 41^{™}, vorzugsweise mit einer mittleren Teilchengröße ≤ 20 µm (z. B. I-HPC LH 32^{™}, I-HPC LH 30^{™} oder I-HPC LH 41™) und einem Hydroxypropylgehalt von 10 bis 13 %, zu verwenden (I-HPC LH 31).

Die Vergleichsversuche zeigen, daß beim Einsatz der bisher bevorzugt zum Einsatz kommenden mikrokristallinen Cellulose mit einer Teilchengröße von ungefähr 50 µm bei der Herstellung von Pellets mit sehr gut wasserlöslichen Wirkstoffen, wie Tramadolhydrochlorid, durch Feuchtextrusion nur Pellets mit einem Wirkstoffgehalt von maximal 40 -45 Gew.% in brauchbarer Ausbeute hergestellt werden können. Agglomeration oder kaum rundbare Extrudatstäbe (dumb-bells) mit hohem Staubanteil je nach Wassermenge bei höheren Wirkstoffgehalten das Ergebnis. Überraschenderweise gelingt es, diesen Nachteil durch die erfindungsgemäß zum Einsatz kommenden Hilfsstoffe zu überwinden.

Der Anteil dieser Hilfsstoffe in der wirkstoffhaltigen Mischung sollte 10 bis 50 Gew.%, vorzugsweise von 20 bis 30 Gew.%, betragen, wobei ein Verhältnis von mikrokristalliner Cellulose zu niedrig substituierter Hydroxypropylcellulose von 4:6 bis 6:4, vorzugsweise 1:1, besonders bevorzugt 5,1:4,9, eingehalten werden sollte.

Die übrigen Prozeßparameter sind dem Fachmann bekannt, wie die Einstellung der Dauer, Geschwindigkeit und Beladung während des Sphäronisierens in Abhängigkeit von Feuchtigkeitsgehalt der Extrudate, die Auswahl des Extrudertyps und die Spheronisationsparameter.

Die nach dem erfindungsgemäßen Verfahren hergestellten Pellets besitzen zunächst keine retardierte Freisetzung für die inkorporierten, hoch wasserlöslichen Wirkstoffe. Sie zeigen aber trotz der hohen Sprengkraft von niedrig substituierter Hydroxypropylcellulose auch nach erfolgter Wirkstofffreisetzung und mehrstündigem Aufenthalt in physiologischen Freisetzungsmedien keinen Zerfall. Sie sind daher ideale Substrate für das Überziehen mit funktionellen Überzügen, wie z. B. Retardüberzügen oder magensaftresistenten Überzügen. Eine Verpressung der überzogenen Pellets zu schnell zerfallenden Pellettabletten, eventuell in Kombination mit nicht überzogenen Pellets als Initialdosis, ist ebenfalls möglich. Der Vorteil der Formulierung liegt dabei in dem Einbau hoher Wirkstoffmengen trotz deren hoher Wasserlöslichkeit. Dadurch lassen sich hohe Dosierungen des eingearbeiteten Wirkstoffes in Form von kleinen Kapseln oder Tabletten verabreichen, die für den Patienten in aller Regel angenehmer einzunehmen sind.

Ein weiterer Gegenstand der Erfindung sind daher auch Verfahren zur Herstellung von Pellets mit retardierten und/oder magensaftresistenten Überzügen, gegebenenfalls zu Tabletten verpreßt oder in Kapseln abgefüllt, indem die erfindungsgemäß hergestellten Pellets nach ihrer Herstellung mit entsprechenden Überzügen versehen werden.

Als Überzugsmaterialien sind alle pharmazeutisch unbedenklichen Überzugsmaterialien geeignet, die dem Fachmann bekannt sind. Vorzugsweise werden natürliche, gegebenenfalls modifizierte oder synthetische Polymere als Überzugsmaterialien verwendet. Es sind dies Polymere, wie z. B. Celluloseether oder Acrylharze. Ganz besonders bevorzugt sind wasserunlösliche oder wasserquellbare Cellulosederivate, wie Alkylcellulose, vorzugsweise Ethylcellulose, oder wasserunlösliche Acrylharze, wie Poly(meth)acrylsäure und/oder deren Derivate, wie deren Salze, Amide oder Ester. Es können auch wasserunlösliche Wachse als Überzugsmaterial verwendet werden.

Diese Materialien sind aus dem Stande der Technik, z. B. Bauer, Lehmann, Osterwald, Rothgang "Überzogene Arzneiformen", Wissenschaftliche Verlagsgesellschaft mbH Stuttgart, 1998, Seite 69 ff., bekannt und werden hiermit als Referenz eingeführt.

Neben den wasserunlöslichen Polymeren und Wachsen können gegebenenfalls zur Einstellung der Freisetzungsrate des Wirkstoffes auch vorzugsweise bis zu 30 Gew.% an nicht retardierenden, vorzugsweise wasserlöslichen Polymeren, wie beispielsweise Polyvinylpyrrolidon oder wasserlösliche Cellulosederivate, wie Hydroxyethylcellulose, Hydroxypropylmethylcellulose oder Hydroxypropylcellulose, und gegebenenfalls in Kombination mit bekannten Weichmachern mitverwendet werden.

Neben dem retardierten Überzug können die Wirkstoff-Zubereitungen noch mit weiteren Überzügen ausgerüstet werden.

Dabei kann beispielsweise ein solcher Überzug aus einem vom Material des retardierenden Überzuges unterschiedlichen Material als nichtretardierende Trennschicht auf der Substratoberfläche aufgebracht sein.

Als Überzugsmaterialien kommen für diese Trennschicht bevorzugt Celluloseether, Polyvidone, Polyacrylate oder auch polymere Naturstoffe in Frage.

Es ist auch möglich, den weiteren Überzug, vorzugsweise über den retardierenden Überzug, aus dem Wirkstoff der Substrate oder aus einem von diesen unterschiedlichen Wirkstoff zu machen, aus dem dieser Wirkstoff unretardiert nach der oralen Verabreichung freigesetzt wird. Durch diesen Mehrschichtenüberzug kann nach der Verabreichung des Präparates sehr rasch eine Initialdosis zur anfänglichen Therapierung zur Verfügung gestellt werden, wobei das Niveau des Wirkstoffes durch die anschließende retardierte Abgabe des Wirkstoffes gehalten werden kann. Als Überzugsmaterialien kommen dafür pharmazeutisch unbedenkliche Materialien in Kombination mit dem Initialwirkstoff, wie beispielsweise Celluloseether, Polyvidone oder Polyacrylate in Frage.

Desweiteren können die Pellets neben dem retardierenden Überzug auch noch Überzüge aufweisen, die sich pH-abhängig auflösen. So kann z. B. erreicht werden, daß zumindest ein Teil der Pellets eines Präparates den Magentrakt ohne Freisetzung passiert und die Wirkstoffe erst im Darmtrakt freigesetzt werden.

### Beispiel 1

Herstellung von Tramadolhydrochloridpellets mit 55 Gew.% Wirkstoffgehalt

| | |
|---|---|
| mikrokristalline Cellulose mit einer mittleren Teilchengröße von 20 µm (Avicel PH 105) | 1150 g |
| | |
| niedrig substituierte Hydroxypropylcellulose (1-HPC LH 31) mittlere Teilchengröße 20 µm | 1100 g |
| Tramadol-HCI | 2750 g |

Der Wirkstoff und die Hilfsstoffe wurden in einem Diosna P25 Granulator zuerst 10 min lang gemischt und anschließend 10 min lang mit 2100 g gereinigtem Wasser granuliert. Das feuchte Granulat wurde in einem Extruder vom Typ NICA E140 mit einer 1 x 2 mm Lochmatritze extrudiert und anschließend bei 900 min⁻¹ und einer Beladung von jeweils 3 kg 10 min lang in einem Spheroniser vom Typ NICA S450 gerundet. Die feuchten Pellets wurden über Nacht im Trockenschrank bei 45°C getrocknet und anschließend abgefüllt.

Die Siebanalyse wurde mit 100 g Pellets in einem Vibrationssiebturm der Firma Fritsch (10 min) und Analysenssieben mit Maschenweiten von 630 µm bis 2000 µm durchgeführt. Die Rückstände auf den einzelnen Siebböden wurden durch Auswiegen bestimmt und die Gewichte der einzelnen Siebfraktionen in Gew.% der Gesamtprobe dargestellt. Die dargestellten Fraktionsanteile ergaben sich jeweils aus n = 3 Siebanalysen.

| Siebfraktion in µm | Gew.% |
|---|---|
| < 800 | 3 |
| 800 - 1250 | 94 |
| 1250 - 1400 | 3 |

Die Ausbeute an runden Pellets mit einer Korngröße von 800 bis 1250 µm beträgt 94 %.

### Beispiel 2:

Herstellung von Tramadolhydrochloridpellets mit 70 Gew.% Wirkstoffgehalt

| | |
|---|---|
| mikrokristalline Cellulose mittlere Teilchengröße 20 µm (Avicel PH 105) | 77,5 g |
| | |
| niedrig substituierte Hydroxypropylcellulose mittlere Teilchengröße 20 µm (1-HPC LH 31) | 72,5 g |
| | |
| Tramadol-HCI | 350,0 g |

Die Herstellung der Pellets erfolgte analog Beispiel 1. Pulvermischungen und Granulation erfolgten aber mit 108 g gereinigtem Wasser im Kenwood Chef Mixer. Es wird mit einer 1,2 x 2,4 mm Lochmatritze extrudiert und bei einer Beladung des Spheronisers von ca. 600 g spheronisiert. Die Siebanalyse der Pellets erfolgte analog Beispiel 1.

| Siebfraktion in µm | Gew.% |
|---|---|
| < 1000 | 1 |
| 1000 - 1600 | 98 |
| (1600 - 2000 | 1 |

Die Ausbeute an runden Pellets mit einer Korngröße von 1000 - 1600 µm beträgt 98 %.

### Beispiel 3:

Herstellung von Tramadolhydrochloridpellets mit 90 Gew.% Wirkstoffgehalt

| | |
|---|---|
| mikrokristalline Cellulose (Emcocel SP 15) mittlere Teilchengröße 15 µm | 27,5 g |
| | |
| niedrig substituierte Hydroxypropylcellulose mittlere Teilchengröße 20 µm (1-HPC LH 31) | 22,5 g |
| | |
| Tramadol-HCI | 450,0 g |

Die Granulation erfolgte mit 70 g gereinigtem Wasser, ansonsten wurden Herstellung und Testung der Pellets analog Beispiel 2 durchgeführt.

| | |
|---|---|
| Siebfraktion in µm | Gew.% |
| < 1000 | 2 |
| 1000 - 1600 | 90 |
| 1600 - 2000 | 8 |

Die Ausbeute an runden Pellets mit einer Korngröße von 1000 - 1600 µm beträgt 90 %.

### Beispiel 4:

Herstellung von Metamizolnatriumpellets mit 80 Gew.% Wirkstoffgehalt

| | |
|---|---|
| mikrokristalline Cellulose (Avicel PH 105) mittlere Teilchengröße 20 µm | 100,0 g |
| | |
| niedrig substituierte Hydroxypropylcellulose mittlere Teilchengröße 10 µm (1-HPC LH 41) | 100,0 g |
| | |
| Metamizol-Na | 800,0 g |

Die Granulation erfolgte mit 200 g gereinigtem Wasser, für die Extrusion wurde eine 1 x 2 mm Lochmatritze verwendet und die Beladung des Spheronisers betrug 1200 g. Ansonsten wurden Herstellung und Testung der Pellets analog Beispiel 2 durchgeführt.

| Siebfraktion in µm | Gew.% |
|---|---|
| < 800 | 2 |
| 800 - 1250 | 95 |
| 1200 - 2000 | 3 |

Die Ausbeute an runden Pellets mit einer Korngröße von 800 - 1250 µm beträgt 95 %.

## Patentansprüche

1. Verfahren zur Herstellung von Pellets enthaltend ≥ 50 Gew.% eines pharmazeutischen Wirkstoffs mit einer Wasserlöslichkeit ≥ 0,5 g/ml durch wässrige Granulation der wirkstoffhaltigen Mischung, Extrusion, Rundung und Trocknung der feuchten Granulate, dadurch gekennzeichnet, daß die wirkstoffhaltige Mischung aus
A) wenigstens 50 Gew.% wenigstens eines Wirkstoffes und mit einer Wasserlöslichkeit von > 0,5 g/ml
B) höchstens 50 Gew.% der Kombination aus
a) einer mikrokristallinen Cellulose mit einer mittleren Teilchengröße von 15 - 20 µm
und
b) einer niedrig substituierten Hydropropylcellulose mit einer mittleren Teilchengröße im Bereich von 10 - 25 µm
besteht,
wobei das Gewichtsverhältnis von a) : b) im Bereich von 4:6 bis 6:4 liegt und in die Mischung nur soviel Wasser eingearbeitet wird, daß diese eine ausreichende Plastizität zur Extrusion und Sphäronisation hat.

2. Verfahren nach Anspruch 1, dadurch gekennzeichnet, daß das Gewichtsverhältnis von a) zu b) 1:1 beträgt.

3. Verfahren nach Anspruch 1 oder 2, dadurch gekennzeichnet, daß als Wirkstoff Tramadolhydrochlorid mit einer Wasserlöslichkeit von > 3,0 g/ml, Metamizol-Na bzw. Diphenhydraminhydrochlorid jeweils mit einer Wasserlöslichkeit von 1 g/ml verwendet wird.

4. Verfahren nach einem oder mehreren Ansprüchen 1 bis 3, dadurch gekennzeichnet, daß Pellets rnit einem Wirkstoffanteil bis zu 90 Gew.% hergestellt werden.

5. Verfahren nach einem oder mehreren Ansprüchen 1 bis 4, dadurch gekennzeichnet, daß die Pellets mit einem Retardüberzug und/oder magensaftresistenten Überzug versehen werden.

6. Verfahren nach einem oder mehreren Ansprüchen 1 bis 5, dadurch gekennzeichnet, daß die Pellets in Kapseln abgefüllt oder zu Tabletten verpreßt werden.

7. Verfahren nach Anspruch 1, dadurch gekennzeichnet, daß die Mischung wenigstens 65 Gew.% der Komponente A) und höchstens 35 Gew.% der Kombination B) enthält.
